# EUROPÄISCHE PATENTSCHRIFT

(11) **EP 2 497 822 B1**
(45) Veröffentlichungstag und Bekanntmachung des Hinweises auf die Patenterteilung: **13.12.2017**
(21) Anmeldenummer: 11001904.9
(22) Anmeldetag: 08.03.2011
(51) Int. Cl.: C12M 1/107, B01F 7/00, B01F 15/00

(54) **Biogasanlagen-Fermenterbehälter mit einer Serviceeinrichtung**
Biogas facility fermenter container with a service device
Récipient de fermentation d'installations de biogaz doté d'un dispositif d'entretien

(43) Veröffentlichungstag der Anmeldung: 12.09.2012
(73) Patentinhaber: Niederbacher, Michael, Dr., 39031 Bruneck (IT)
(72) Erfinder: Niederbacher, Michael, Dr., 39031 Bruneck (IT)
(74) Vertreter: Liebl, Thomas

(56) Entgegenhaltungen:
- DE-A1- 19 951 959
- DE-A1-102009 031 177
- DE-U1-202007 007 060

## Beschreibung

Die Erfindung betrifft einen Biogasanlagen-Fermenterbehälter mit einer Serviceeinrichtung gemäß dem Oberbegriff des Anspruchs 1. Um an einem durch eine Rühreinheit gebildeten Tauchgerät eines Biogasanlagen-Fermenterbehälters Wartungs- und Servicearbeiten durchführen zu können, ohne dass der zuständige Monteuer ständig den im Fermenterbehälter-Innenraum gebildeten Fermentationsgasen ausgesetzt ist, ist es aus der DE 197 14 342 C1 bereits bekannt, einen Dom als gasdichte Abdeckung über einer deckenseitigen Rührwerköffnung anzubringen. Ein oberer Stützstangenbereich einer Stützstange als Führungsmast, entlang dem das Tauchgerät höhenverstellbar geführt ist, ragt hierbei mit der Betätigungseinrichtung über eine Dachwand des Domes hinaus. Die Rühreinheit selbst kann mittels einer Höhenverstelleinrichtung aus dem Fermenterbehälter-Innenraum in den Innenraum des Domes bewegt werden und ist dort durch wenigstens eine gasdicht verschließbare Domöffnung zugänglich. Damit sollen sowohl einfache als auch umfangreiche Reparatur- bzw. Wartungsarbeiten vom Monteur nicht mehr im Fermenterbehälter-Innenraum selbst durchgeführt werden, sondern bequemer und einfacher und damit auch preisgünstiger von außen durch die Domöffnung hindurch durchgeführt werden können. Da bei einem derartigen Aufbau aber nach wie vor Gas aus der Gasphase oberhalb des zu vergärenden flüssigen Substrates durch die Rührwerköffnung hindurch in den Innenraum des Doms gelangen kann, ist bzw. sind die Monteure nach wie vor den Fermentationsgasen ausgesetzt. Um dies möglichst zu vermeiden, schlägt die DE 197 14 342 C1 weiter vor, die Rührwerköffnung von der nicht verschlossenen Domöffnung her mit auf den öffnungsseitigen Auflagekanten aufliegenden Brettern abzudecken, die weiter mit einer Gummimatte abgedeckt sind, welche Gummimatte eine dem Stützstangenquerschnitt entsprechende Aussparung aufweist. Eine derartige Abdichtung der Rührwerköffnung ist unpraktikabel und sehr zeitaufwändig, da hier mit zusätzlichen Brettern und Gummimatten hantiert werden muss. Diese Maßnahme zur Abdichtung der Rührwerköffnung zur Gasphase des Fermenterbehälters hin wird daher im praktischen Einsatzfall regelmäßig nicht verwendet, so dass die Monteure in unerwünschter Weise nach wie vor dem Gas ausgesetzt sind.

Das zuvor Gesagte gilt in gleicher Weise für eine Serviceeinrichtung, wie sie in der EP 2 174 704 A1 beschrieben ist, bei der im normalen Tauchgerätebetrieb eine Deckenöffnung des Fermenterbehälters durch eine Podestplatte und ein angrenzendes, die Ebene der Podestplatte überragendes Mastgehäuse gasdicht abgedeckt ist. Im Mastgehäuse ist ein Mastoberteil eines vertikalen Führungsmastes einschließlich einer Höhenverstelleinrichtung mit einer Seilrolle, einem zugeordneten Zugseil und gegebenenfalls einer Getriebeeinheit dergestalt eingehaust, dass das Mastgehäuse Seltenwände aufweist, nach unten zum Fermenterbehälter offen ist und oben durch eine Gehäuseabdeckung verschlossen ist, die ein Stützlager für das obere Mastende enthält. Zumindest eine Seitenwand des Mastgehäuses ist in unmittelbarer Nähe des Mastoberteils angeordnet und als bedarfsweise öffenbare Wartungswand ausgeführt. Im unteren Bereich der öffenbaren Wartungswand grenzt ebenfalls eine bedarfsweise offenbare Podestplatte an, so dass für eine Wartungsbereitstellung des Tauchgerätes, die Wartungswand und die Podestplatte zu öffnen sind und das Tauchgerät mittels der Seilrolle und dem Zugseil nach oben über die Ebene der geöffneten Podestplatte aus dem Fermenterbehälter ausgehoben werden kann, so dass das Tauchgerät im Bereich vor der geöffneten Wartungswand frei zugänglich ist. Bei einem derartigen Aufbau einer Serviceeinrichtung besteht jedoch wiederum das Problem, dass bei geöffneter bzw. abgenommener Wartungswand und Podestplatte die Fermentationsgase über die Decken- bzw. Serviceöffnung ungehindert entweichen können und damit eine gesundheitliche Beeinträchtigung für den am herausgehobenen Tauchgerät arbeitenden Monteur darstellen.

Um die eben beschriebenen Nachteile zu vermeiden ist es weiter aus der gattungsbildenden DE 10 2009 031 177 A1 bereits bekannt, eine Serviceeinrichtung mit einer Serviceeinheit und einem Servicebereich vorzusehen, welche Serviceeinheit beispielsweise durch einen domartigen Serviceschacht, wie vorstehend beschrieben, gebildet sein kann. An dieser Serviceeinheit ist eine bewegliche Dichtungseinrichtung angeordnet, die zwischen einer Ruhestellung und einer Dichtungsstellung hin und her verlagert werden kann, wobei die Dichtungseinrichtung geeignet sein soll, den Servicebereich von dem Gasbereich in einer Dichtungsstellung im Wesentlichen gasdicht zu trennen. Die Dichtungseinrichtung ist hier durch eine Dichtungswand aus einem flexiblem Material gebildet, beispielsweise faltenbalgartig oder teleskopartig verlagerbar ausgebildet, wobei weiter ein Führungsseil vorgesehen ist, mit welchem die Dichtungseinrichtung zwischen der Ruhestellung und der Dichtungsstellung hin und her bewegbar ist. In der Dichtungsstellung soll sich die Dichtungseinrichtung von der Serviceeinrichtung ausgehend nach unten erstrecken und auf dem zu vergärenden flüssigen Substrat aufliegen bzw. mit dem Rand ein wenig in den Fermentationsbereich eintauchen. Ein derartiger beweglicher Gasvorhang ermöglicht zwar Service- bzw. Wartungsarbeiten am aus dem Fermenterbehälter-Innenraum herausgehobenen Tauchgerät, ist jedoch relativ aufwändig in der Bedienung, um Gasdichtheit herzustellen und darüber hinaus auch sehr fehleranfällig bzw. störanfällig, weil sich Feststoffe, zum Beispiel in Verbindung mit Schwimmschichten, sowie generell Ablagerungen, zum Beispiel durch Schwefel, an dem Dichtungsvorhang bzw. der damit verbundenen Hebemechanik festsetzen bzw. anlagem können und damit die ordnungsgemäße Funktion des Dichtungsvorhangs beeinträchtigen können. Des Weiteren besteht die Gefahr, dass die flexible Wand des Dichtungsvorhangs Im abgesenkten Zustand und damit in ihrer Dlchtungsstellurig, insbesondere in Verbindung mit niedrigen Füllstandshöhen, keine ausreichende Länge aufweist, um in das flüssige, zu vergärende Substrat einzutauchen und daher das Gas in unerwünschter Weise über die Serviceöffnung entweichen kann. Wird zur Vermeidung eines solchen Falles eine ausreichende Länge des Dichtungswandmaterials vorgehalten, besteht wiederum in Verbindung mit hohen Flüssigkeits- bzw. Substratfüllständen die Gefahr, dass das Dichtungselement aufschwimmt und gegebenenfalls sogar eine Beeinträchtigung des in der Nähe desselben angeordneten Rührwerks zu befürchten ist. Ein weiterer Nachteil besteht darin, dass durch den Druckunterschied zwischen der Innenseite des flexiblen Gasvorhangs und dessen Außenbereich die Gefahr besteht, dass sich der Gasvorhang in den Servicebereich verbiegen kann.
Aufgrund der Flexibilität der Wände der verlagerbaren Dichtungseinrichtung besteht zudem im rauen Fermentationsbetrieb nach wie vor die Gefahr, dass das Dichtungselement keine funktionssicher abdichtende Einrichtung ausbildet und nach wie vor eine unerwünscht hohe Gasmenge über eine derartige Gasschleuse aus der Serviceöffnung austreten kann.
Es ist daher Aufgabe der vorliegenden Erfindung einen Biogasanlagen-Fermenterbehälter mit einer Serviceeinrichtung zur Verfügung zu stellen, mittels dem Wartungs-und Servicearbeiten, insbesondere an einem Tauchgerät, wie beispielsweise einem Tauchrührgerät oder einer Tauchpumpe, auf einfache und zuverlässige Weise ohne nennenswerte Beeinträchtigung des Monteurs durch aus dem Fermenterbehälter entweichendes Gas durchgeführt werden kann.
Diese Aufgabe wird bezüglich des Biogasanlagen-Fermenterbehälters gelöst mit den Merkmalen des Patentanspruches 1. Vorteilhafte Ausgestaltungen hierzu sind jeweils Gegenstand der darauf rückbezogenen Unteransprüche.

Gemäß Anspruch 1 wird ein Biogasanlagen-Fermenterbehälter mit einer Serviceeinrichtung vorgeschlagen, die eine mittels einer Abdeckeinrichtung gasdicht verschließbare deckenseitige Serviceöffnung aufweist, durch die ein an einem Führungsmast höhenverstellbar geführtes Tauchgerät, insbesondere ein Tauchrührgerät oder eine Tauchpumpe, für Wartungs- und Servicearbeiten im Wesentlichen gasdicht aus dem Fermenterbehälter heraus und wieder in den Fermenterbehälter hinein bewegbar ist. Um die Serviceöffnung herum ist ein in der Dichtstellung schachtartig nach unten in den Fermenterbehälter-Innenraum einragendes Dichtelement angeordnet, das wenigstens bei Wartungs- und Servicearbeiten in das im Fermenterbehälter aufgenommene, zu vergärende flüssige Substrat eintaucht und den von dem Dichtelement ringförmig umschlossenen Gasphasenbereich unterhalb der öffenbaren Serviceöffnung sowie oberhalb des Substrates gasdicht von dem restlichen Gasphasenbereich des Fermenterbehälter-Innenraums abtrennt. Erfndungsgemäß ist vorgeschlagen, dass das Dichtelement durch einen starren, nicht verlagerbaren und unflexiblen sowie dauerhaft von der Serviceöffnung in Richtung Fermenterbehälter-Innenraum abragenden rohrstutzenartigen Kragen gebildet ist. Ein solcher starrer Kragen kann zum Beispiel aus einem stabilen Material, zum Beispiel einem Metall, höchst bevorzugt aus einem korrosionsbeständigen Edelstahlmaterial, hergestellt sein und bringt den Vorteil mit sich, dass mit einem derartigen starren, nicht verlagerbaren Kragen als Dichtelement auf einfache Weise sichergestellt werden kann, dass durch die fehlende Verlagerungsmöglichkeit des Dichtelementes Störfälle bei der Abdichtung durch eine fehlerhafte oder gestörte Dichtelementverlagerung bereits von vorneherein ausgeschlossen sind. Zudem ist durch die starre, stabile Ausbildung des rohrstutzenartigen Kragens sichergestellt, dass dieser nicht überdrückt bzw. weggedrückt werden kann und dadurch gegebenenfalls eine Gefahr für einen Rührbetrieb darstellt. Mit einer erfindungsgemäßen Lösung, bei der das Dichtelement durch einen starren, nicht verlagerbaren und unflexiblen sowie dauerhaft in Richtung Fermenterbehälter-Innenraum von der Serviceöffnung abragenden rohrstutzenartigen Kragen ausgebildet ist, wird somit auf einfache und vor allem auch auf funktionssichere Weise eine zuverlässige Abdichtung des Wartungs- und Arbeitsbereiches bei geöffneter Serviceöffnung und aus dem Fermenterbehälter-Innenraum herausgehobenen Tauchgerät möglich.

Um auf einfache Weise sicherzustellen, dass mittels dem starren, rohrstutzenartigen Kragen eine zuverlässige Abdichtung des Servicebereiches erzielt wird, ist gemäß einer besonders bevorzugten Ausgestaltung der erfindungsgemäßen Lösung vorgesehen, dass der Serviceeinrichtung weiter eine Füllstands-Messeinrichtung zugeordnet ist oder eine solche aufweist, mittels der wenigstens bei Wartungs- und Servicearbeiten der Füllstand des Substrates im Fermenterbehälter erfasst und ein entsprechendes Füllstands-Messsignal als Höhenistwert-Signal erzeugt wird, wobei weiter eine Steuereinrichtung vorgesehen ist, der das Füllstands-Messsignal zugeführt wird und die bei einem erfassten geringen Füllstand, bei dem der Kragen nicht in das Substrat eintaucht, wenigstens eine Förder- und/oder Pumpeinrichtung der Biogasanlage dergestalt ansteuert, dass der Füllstand des Substrates im Fermenterbehälter auf ein Niveau angehoben wird, bei dem der Kragenbereich in das Substrat eintaucht. Mit einer derartigen Füllstandskontrolle wird somit auf einfache und funktionssichere Weise zuverlässig sichergestellt, dass wenigstens bei Wartungs- und Servicearbeiten der Füllstand des zu vergärenden flüssigen Substrats eine solche ausreichende Höhe aufweist, dass der starre, rohrstutzenartige Kragen in einem definierten, vorgegebenen Maße in das Substrat eintaucht, um eine zuverlässige und funktionssichere Abdichtung zur Verfügung zu stellen.

Konkret kann hierbei die Füllstands-Messvorrichtung wenigstens einen Sensor aufweisen, mittels dem der aktuelle Füllstand des Substrates im Fermenterbehälter erfasst und als Höhen-Istwert-Signal der Steuereinrichtung zugeführt werden kann, die wenigstens aus diesem Höhen-Istwert-Signal und dem vorgegebenen, bekannten Wert für die Einragtiefe des Kragens in den Fermenterbehälter-Innenraum ermittelt, ob der Kragen in das Substrat eintaucht. Ermittelt die Steuereinrichtung einen solchermaßen geringen Füllstand, bei dem der Kragen nicht in das Substrat eintaucht, wird die wenigstens eine Förder- und/oder Pumpeinrichtung von der Steuereinrichtung solange angesteuert, bis der wenigstens eine Sensor ein Höhenistwert-Signal liefert, das einem Füllstands-Sollwert und damit einem Füllstand des Substrates im Fermenterbehälter entspricht, bei dem der Kragen in einer gewünschten Weise in das Substrat eintaucht. Eine derartige Füllstands-Messvorrichtung mit Steuereinrichtung und Sensortechnik ist auf einfache Weise realisierbar und zudem wenig fehler- und störanfällig, was ebenfalls hilft, auf einfache und funktionssichere sowie bauteiltechnisch preiswerte und einfache Weise eine zuverlässige Abdichtung des Servicebereichs bei Wartungs- und Servicearbeiten zur Verfügung zu stellen.

Grundsätzlich gibt es unterschiedliche Möglichkeiten, den Füllstand im Fermenterbehälter ansteigen zu lassen. Beispielsweise kann hierzu die wenigstens eine Förder- und/oder Pumpeinrichtung durch wenigstens eine, das zu vergärende Substrat aus dem Fermenterbehälter abpumpende Abpumpeinrichtung gebildet sein, die zur Anhebung des Füllstandes von der Steuereinrichtung für eine definierte Zeitdauer abgeschalten wird. Eine derartige Abpumpeinrichtung kann zum Beispiel in Verbindung mit einem Nachfermenter oder dergleichen vorgesehen sein. Alternativ oder zusätzlich dazu kann die wenigstens eine Förder- und/oder Pumpeinrichtung durch wenigstens ein, zu vergärendes flüssiges Substrat in den Fermenterbehälter pumpende bzw. förderende Pumpeinrichtung gebildet sein, die zur Anhebung des Füllstandes, gesteuert von der Steuereinrichtung, Substrat aus einem vor- und/oder nachgeschalteten Behälter in den Fermenterbehälter pumpt. Anstelle von flüssigem, zu vergärendem Substrat könnte grundsätzlich auch ein anderes flüssiges Medium in den Fermenterbehälter gepumpt werden, solange dies mit der Biologie der Fermentation im Fermenterbehälter-Innenraum vereinbar ist. An dieser Stelle sei ausdrücklich erwähnt, dass vorliegend unter dem Begriff zu vergärendes flüssiges Substrat jedwede zu vergärende, flüssige Biomasse bzw. Biomassemischung verstanden wird, die zur Erzeugung von Biogas einem Fermentationsprozess unterzogen werden kann.

Die Serviceöffnung selbst kann grundsätzlich auf unterschiedlichste Art und Weise ausgebildet sein, zum Beispiel rund oder mehreckig, insbesondere rechteckförmig, wobei gemäß einer besonders bevorzugten Ausgestaltung vorgesehen ist, dass die Serviceöffnung Bestandteil einer deckenseitig angeordneten Podestplatte ist, von der der die Serviceöffnung ringförmig umschließende Kragen nach unten abragt. Auch die Podestplatte, die begehbar ausgebildet ist, wird aus einem Metall hergestellt, höchst bevorzugt aus einem Edelstahimaterial. Besonders bevorzugt ist in diesem Zusammenhang eine Ausgestaltung, bei der die Podestplatte und der Kragen aus einem gleichen Material ausgebildet sind. Der Kragen ist integraler Bestandteil der Podestplatte und mit dieser materialeinheitlich und/oder einstückig verbunden sein, insbesondere durch aus der Podestplatte herausgebogene Laschen gebildet sein. In Verbindung mit einem Kragen, der durch ein separates Bauteil hergestellt ist, kann insbesondere vorgesehen sein, dass dieser an seinem der Podestplatte zugewandten freien Endbereich einen im Wesentlichen randseitig umlaufenden bzw. wenigstens bereichsweise randseitig umlaufenden Flanschbereich aufweist, über den die Anbindung an die Podestplatte, um die Serviceöffnung herum, erfolgt. Beispielsweise kann auch vorgesehen sein, dass die Podestplatte auf der dem Fermenterbehälter-Innenraum abgewandten Seite eine dem Randflanschbereich des Kragens entsprechend zugeordnete Ausnehmung bzw. Einsenkung aufweist, in der der Randflanschbereich oberflächenbündig mit der Podestplatte und/oder formschlüssig einliegt. Die Festlegung des Kragens kann dann zum Beispiel stoffschlüssig oder formschlüssig bzw. auch stoff- und formschlüssig im Randflanschbereich des Kragens erfolgen.

Es versteht sich, dass die Serviceöffnung und der Kragen grundsätzlich unterschiedliche Geometrien aufweisen können, solange sichergestellt ist, dass der Kragen die Serviceöffnung vollständig umschließt. Besonders bevorzugt ist jedoch eine Ausgestaltung, bei der der Kragen zumindest im unmittelbaren Angrenzungsbereich an die Serviceöffnung eine der Geometrie der Serviceöffnung entsprechende Geometrie aufweist, zum Beispiel eine rechteckförmige Geometrie oder dergleichen. Es ist vorgesehen, dass sich an die Podestplatte eine Behälterseitenwand, und ein Foliendach des Fermenterbehälters anschließt. Die Podestplatte kann zum Beispiel in eine Betondecke oder dergleichen starre und unflexible Behälterwand, insbesondere Deckenwand integriert sein. In diesem Fall kann dann die Podestplatte durch entsprechende randseitige Auflager unmittelbar von der stabilen Behälterwand bzw. Deckenwand abgestützt und gehaltert werden. Die Anbindung des Podestplatten-Randkantenbereiches mittels einer gasdichten AnlageVerbindung an einer Behälterseitenwand, insbesondere an einer Behälterdeckenwand, erfolgt dabei vorzugsweise mittels einer oder mehrerer Schraubverbindungen. Die Podestplatte kann aber auch Bestandteil eines Fermenterbehälters sein, der mit einem Foliendach ausgestattet ist. Dementsprechend weist dann die Podestplatte einen Foliendach-Anschlussbereich auf, an dem das Foliendach, insbesondere ein Foliendach-Randbereich, gasdicht angebunden ist. Zur gasdichten Anbindung kann insbesondere eine Klemmverbindung vorgesehen sein, und zwar eine Klemmverbindung zwischen einem Klemmbereich, insbesondere einem Randflansch der Podestplatte und einer Klemmleiste, die mit dem Klemmbereich bzw. dem Randflansch lösbar verbunden bzw. verklemmt werden kann. Im Falle der Integration der Podestplatte in einen Fermenterbehälter mit einem Foliendach ist bevorzugt vorgesehen, dass diese Podestplatte mittels einer Abstützeinrichtung, insbesondere mit einem Abstützgestell, von unten her abgestützt ist. Die Abstützeinrichtung selbst ist dann bevorzugt wandseitig am stabilen Fermenterbehälter-Innenraumwandbereich angebunden und festgelegt.

Die die Serviceöffnung gasdicht verschließende Abdeckeinrichtung kann zum Beispiel lediglich durch einen Deckel gebildet sein. Alternativ oder zusätzlich dazu kann die die Serviceöffnung gasdicht verschließende Abdeckeinrichtung aber auch durch ein von der Serviceöffnung weg nach oben ragendes Gehäuse, das einen Aufnahmeraum für wenigstens einen oberen Teilbereich des Führungsmastes ausbildet, gebildet sein. Dadurch ergeben sich erhebliche konstruktive Freiheiten in Verbindung mit der Ausgestaltung der für den jeweiligen Einsatzfall geeigneten Abdeckeinrichtung. Das Gehäuse kann zum Beispiel domartig ausgebildet sein und einen oberhalb der Serviceöffnung von außen zugänglichen Aufnahmeraum aufweisen, in den das Tauchgerät mittels einer Höhenverstelleinrichtung hinein bewegbar ist. Alternativ dazu kann das Gehäuse aber auch lediglich als Mastgehäuse ausgebildet sein, das lediglich einen oberen Teilbereich des Führungsmastes einhaust, wobei eine Mastgehäuse-Seitenwand und eine serviceöffnungsseitige Abdeckplatte entfernt werden können, um die Serviceöffnung freizugeben und das Tauchgerät entsprechend aus dem Fermenterbehälter herauszuheben bzw. wieder in diesen abzusenken. Der Führungsmast selbst ist bevorzugt abdeckeinrichtungsseitig gelagert, wobei weiter bevorzugt vorgesehen ist, dass die Bauteile einer Höhenverstelleinrichtung zum Anheben des Tauchgerätes bzw. zum Absenken des Tauchgerätes, wie beispielsweise ein Zugseil mit Seiltrommel sowie gegebenenfalls einer Getriebeeinheit, im Bereich der Abdeckeinrichtung selbst angeordnet und/oder gelagert sind. In diesem Zusammenhang kann insbesondere vorgesehen sein, dass im Falle eines Gehäuses als Abdeckeinrichtung oder als Bestandteil der Abdeckeinrichtung die Bauteile der Höhenverstelleinrichtung wenigstens zum Teil innerhalb des Gehäuses angeordnet sind. Die Betätigungseinrichtung der Höhenverstelleinrichtung, zum Beispiel eine Handkurbel oder dergleichen, kann dabei auf jeden Fall gut von außen zugänglich angeordnet sein.

Die vorstehend erläuterten und/oder in den Unteransprüchen wiedergegebenen vorteilhaften Aus- und Weiterbildungen der Erfindung können dabei - außer zum Beispiel in den Fällen eindeutiger Abhängigkeiten und unvereinbarer Alternativen - einzeln oder aber auch in beliebiger Kombination miteinander zur Anwendung kommen.

Die Erfindung und ihre vorteilhaften Aus- und Weiterbildungen sowie deren Vorteile werden nachfolgend anhand von Zeichnungen näher erläutert.

Es zeigen, jeweils in einer schematischen Prinzipskizze:
- Fig. 1: einen Querschnitt durch einen Teilbereich eines Biogasanlagen-Fermenterbehälters mit unterhalb eines rohrstutzenartigen Kragens befindlichem Füllstand des flüssigen, zu vergärenden Substrates,
- Fig. 1a, 1b: ergänzende Schnittdarstellungen entlang der um 90° gedrehten Linie Z-Z der Fig. 1,
- Fig. 2: eine schematische Darstellung entsprechend Fig. 1 mit zu Wartungs- und Servicearbeiten am Tauchmotorrührgerät dergestalt angehobenem Füllstand, dass der rohrstutzenartige Kragen in die flüssige, zu vergärende Substanz eintaucht,
- Fig. 3a: eine schematische, perspektivische Draufsicht auf eine erfindungsgemäße Podestplatte mit Serviceöffnung und nach unten abragendem rohrstutzenartigen Kragen,
- Fig. 3b: schematisch eine Draufsicht auf die Podestplatte der Fig. 3a,
- Fig. 3c: schematisch eine Schnittansicht entlang der Linie A-A der Fig. 3b,
- Fig.3d: schematisch eine Schnittansicht entlang der Linie B-B der Fig. 3b,
- Fig. 3e: schematisch eine Rückansicht der Podestplatte entsprechend dem Pfeil R in Fig. 3b, und
- Fig. 3f: eine Seitenansicht der Podestplatte entsprechend dem Pfeil S in der Fig. 3e.

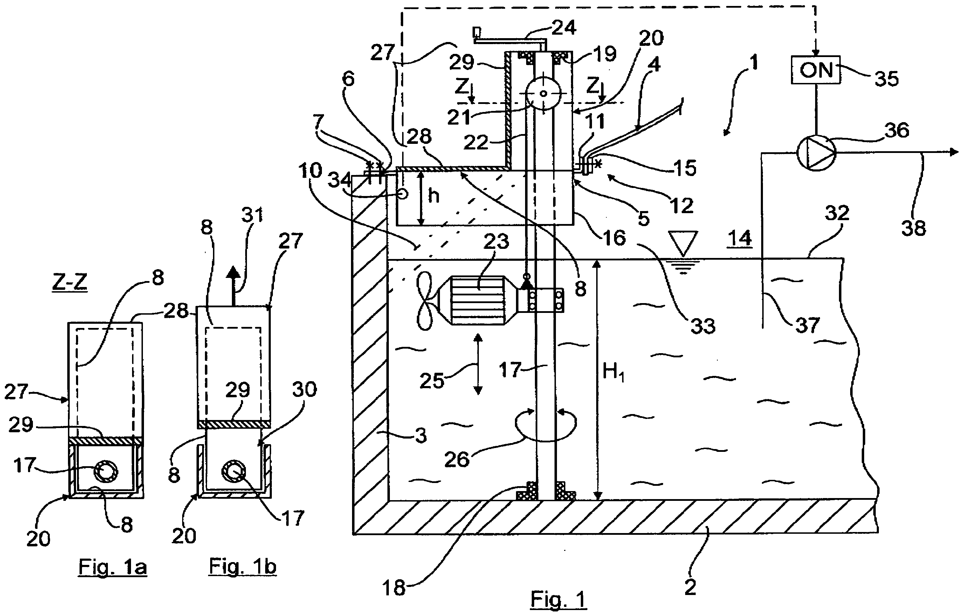

In der Fig. 1 ist schematisch ein Querschnitt durch einen Teilbereich eines erfindungsgemäßen Biogasanlagen-Fermenterbehälters 1 gezeigt, der sowohl ein Vor-, als auch ein Haupt-, als auch ein Nach-Fermenterbehälter sein kann. Dieser Fermenterbehälter weist eine Bodenwand 2 sowie eine beispielsweise kreiszylindrische Seitenwand 3 auf und ist an seiner Oberseite und damit deckenseitig mit einem hier lediglich schematisch und prinzipiell dargestellten Foliendach 4 abgedeckt. Insofern ist der Fermenterbehälter 1 herkömmlicher Bauart. In der hier in der Fig. 1 gezeigten Ausgestaltung ist an einem Fermenterbehälterrandbereich, der Seitenwand 3 zugeordnet, eine Podestplatte 5 angeordnet, die einen der Seitenwand 3 zugeordneten Podestplatten-Randkantenbereich 6 aufweist, mittels dem die Podestplatte 5 hier beispielhaft von oben her auf der Seitenwand 3 aufliegt und dort mittels der hier lediglich schematisch dargestellten Schraubverbindungen 7 lösbar festgelegt ist. Zur Herstellung der Gasdichtheit kann hier zum Beispiel ein nicht gezeigtes Dichtelement im Auflagebereich des Podestplatten-Randkantenbereichs 6 auf der Seitenwand 3 angeordnet und vorgesehen sein.

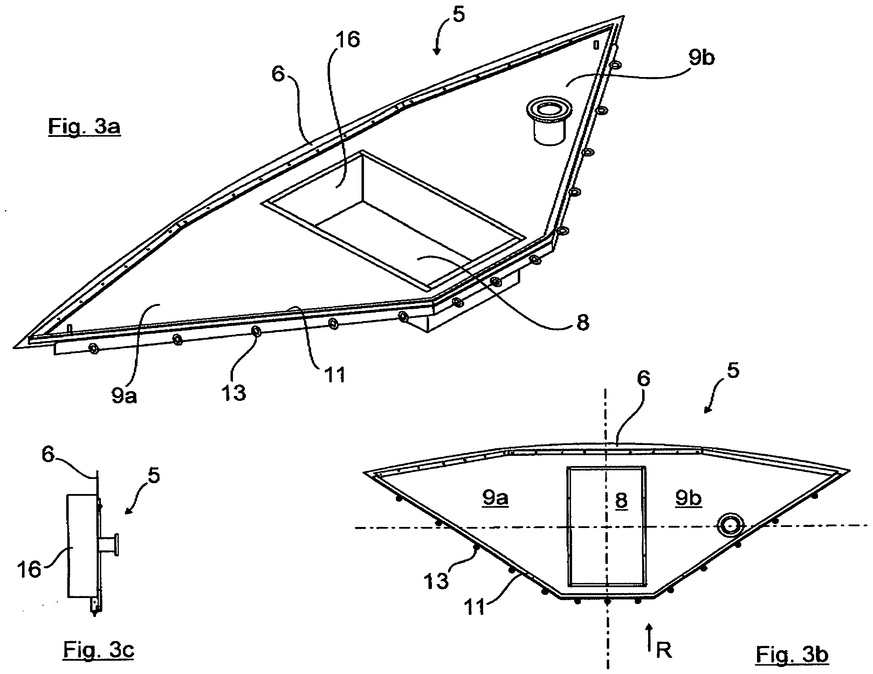

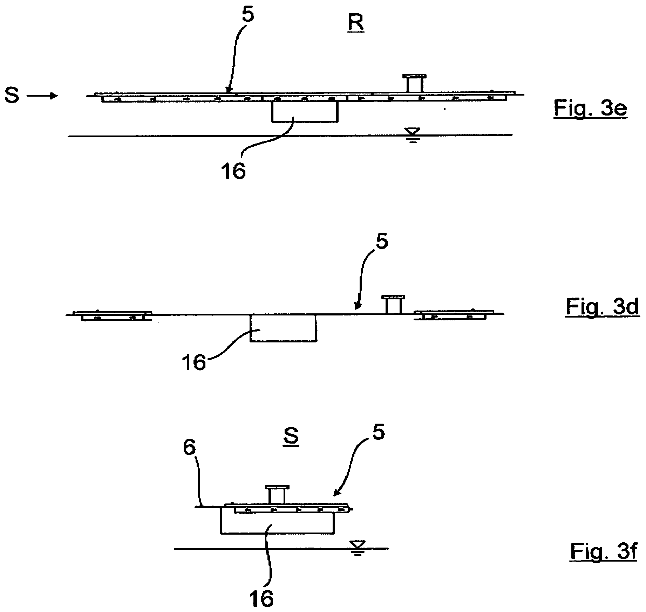

Wie dies insbesondere aus der Fig. 3a und der Fig. 3b ersichtlich ist, weist die Podestplatte 5 in etwa mittig und zentral eine hier beispielhaft rechteckförmige Serviceöffnung 8 auf, an die sich zu beiden Seiten hin flügelartige Podestplattenbereiche 9a, 9b anschließen. An diesen flügelartigen Podestplattenbereichen 9a, 9b greifen beispielsweise ein oder mehrere in der Fig. 1 lediglich schematisch strichpunktiert dargestellte Stützen 10 eines Abstützgestells von unten her an, welche Stützen 10 weiter am stabilen Seitenwandbereich 3 fest angebunden sind, so dass die Stützen 10 des hier nicht im Detail dargestellten Abstützgestells die Podestplatte 5 in der in der Fig. 1 gezeigten Position halten.

Auf der dem Podestplatten-Randkantenbereich 6 gegenüberliegenden Seite der Podestplatte 5 erstreckt sich über die gesamte Länge dieser Seite ein im Wesentlichen nach oben abstehender Randflansch 11 als Klemmbereich, zwischen dem und einer hier in der Fig. 3a nicht, sondern lediglich äußerst schematisch in der Fig. 1 gezeigten Klemmleiste 15, die beispielsweise mit dem Randflansch 11 mittels mehrerer Schraubverbindungen verbunden werden kann, ein Abschnitt des Foliendaches 4 gasdicht verklemmt ist. Die Klemmleiste 15 kann hier sowohl einstückig als auch mehrteilig ausgebildet sein.

In diesem Foliendach-Anschlussbereich 12 ist ferner, was lediglich aus der Fig. 3a ersichtlich ist, unterhalb des Randflansches 11 eine Mehrzahl von voneinander in Randflansch-Längsrichtung beabstandeten Ösen 14 angeordnet, durch die zum Beispiel hier nicht dargestellte Seile zum Festlegen bzw. Abspannen des Foliendaches 4 hindurchgefädelt und/oder festgebunden werden können.

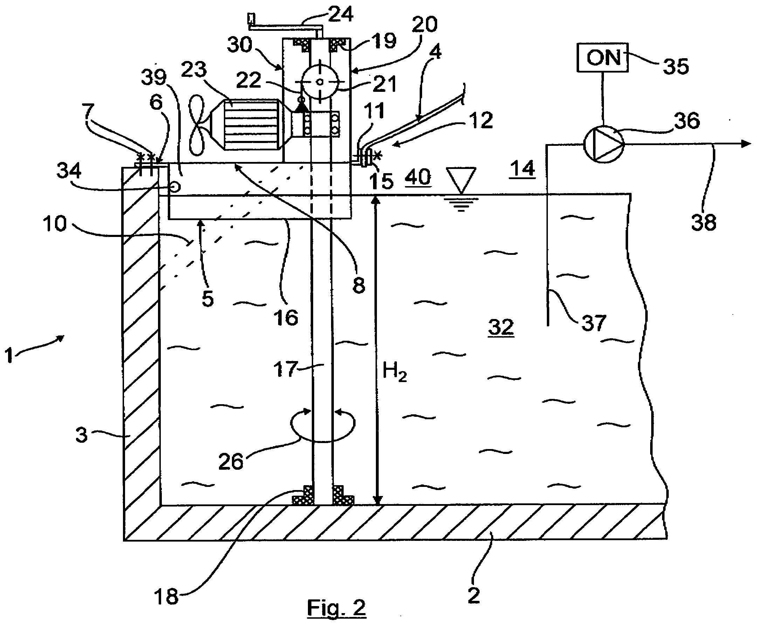

Wie dies weiter insbesondere den Fig. 3a bis 3f entnommen werden kann, ist um die Serviceöffnung 8 herum ein starrer, im montierten Zustand der Podestplatte 5 (Fig. 1 und 2) schachtartig nach unten in den Fermenterbehälter-Innenraum 14 einragender, nicht verlagerbarer und unflexibler rohrstutzenartiger Kragen 16 angeordnet, der, wie dies beispielhaft in der Fig. 3a gezeigt ist, zum Beispiel durch ein separates Bauteil aus einem zum Beispiel Edelstahlmaterial hergestellt sein kann und dann mit der Podestplatte 5 verschraubt bzw. auch verschweißt sein kann. Im hier gezeigten Beispielfall weist der Kragen 16 eine im Wesentlichen identische Geometrie wie die Serviceöffnung 8 auf und schließt somit unmittelbar an den Randkantenbereich der Serviceöffnung 8 nach unten hin an. Im montierten Zustand (Fig. 1) beträgt die Einragtiefe h des Kragens 16 beispielsweise ca. 50cm oder dergleichen.

Wie dies aus den Fig. 1 und 2 weiter ersichtlich ist, ist durch die Serviceöffnung 8 ferner ein Führungsmast 17 geführt, der in Stützlagern 18 bzw. 19 gelagert, insbesondere verdrehbar gelagert ist. Das Stützlager 18 ist dabei bodenseitig an der Bodenwand 2 des Fermenterbehälters 1 angeordnet, während das Stützlager 19 in einem die Serviceöffnung 8 nach oben abdeckenden Mastgehäuse 20 angeordnet ist. An dem in das Mastgehäuse 20 einragenden oberen Teil des Führungsmastes 17 ist hier eine Seiltrommel 21 verdrehbar gelagert, mittels der ein mit einem Tauchmotorrührgerät 23 verbundenes Seil 22 bei einer Betätigung der hier außerhalb des Mastgehäuses 20 angeordneten, lediglich beispielhaft dargestellten Handkurbel 24 so auf- und abgewickelt werden kann, dass das Tauchmatarrührgerät entsprechend des Doppelpfeils 25 entlang des Führungsmastes 17 nach oben bzw. unten verlagert werden kann. Die Verdrehbarkeit des Führungsmastes 17 ist hier durch den Doppelpfeil 26 lediglich schematisch gezeigt.

An dieser Stelle sei ausdrücklich erwähnt, dass die Höhenverstellvorrichtung mitsamt Seiltrommel 21, Seil 22 und Handkurbel 24 hier lediglich beispielhaft und schematisch sowie stellvertretend für sämtliche möglichen Höhenverstelleinrichtungen steht; beispielsweise kann eine Höhenverstellung auch mittels einer Spindelanordnung erfolgen bzw. kann anstelle einer Handkurbel auch eine elektrische, pneumatische oder hydraulische Betätigung erfolgen. Des Weiteren versteht es sich von selbst, dass eine Bewegungsübertragungseinrichtung vorhanden sein muss, die die Betätigung der Handkurbel 24 in eine entsprechende Drehbewegung der Seiltrommel 21 umsetzt.

Wie dies aus der schematischen Schnittansicht Z-Z der Fig. 1 a und der Fig. 1 b hervorgeht, umschließt das Mastgehäuse 20 den Führungsmast 17 nur bereichsweise bzw. im hier gezeigten Beispielfall lediglich U-förmig, wobei an dieser Stelle bemerkt werden soll, dass der Schnittverlauf Z-Z in der Fig. 1 aus Übersichtlichkeitsgründen um 90° versetzt eingezeichnet worden ist.

Um die in der Fig. 1a und Fig. 1b teilweise strichliert und teilweise mit durchgezogenen Linien dargestellte Serviceöffnung 8 jedoch vollständig abdecken zu können ist des Weiteren eine hier lediglich äußerst schematisch und beispielhaft dargestellte L-förmige Abdeckplatte 27 vorgesehen, die zum einen mit einem ersten L-Schenkel 28 den Rest der vom Mastgehäuse 20 nicht überdeckten Serviceöffnung 8 überdeckt und mit einem zweiten L-Schenkei 29 eine mastgehäuseseitige Wartungsöffnung 30 abdeckt, damit kein Gas über die Serviceöffnung 8 bzw. das Mastgehäuse 20 in die Umgebung entweichen kann. Mastgehäuse 20 und Abdeckplatte 27 bilden hier somit zusammen eine Abdeckeinrichtung aus, mittels der der Bereich oberhalb der Serviceöffnung gasdicht abgedeckt werden kann. Die Abdeckplatte 27 kann dabei mit der Podestplatte im Auflagebereich entsprechend kraftschlüssig mittels einer oder mehrerer Schraubverbindungen verbunden sein. Das Gleiche gilt für die Anbindung der Abdeckplatte 27 mittels des zweiten L-Schenkels 29 an entsprechenden Auflagern der U-Schenkelstirnseiten des Mastgehäuses 20. Die L-Schenkel 28, 29 können grundsätzlich auch durch separate Bauteile gebildet und die Abdeckplatte 27 dementsprechend mehrteilig aufgebaut sein.

In der Fig. 1b ist mit dem Pfeil 31 der Zustand gezeigt, bei dem die Abdeckplatte 27 von dem Mastgehäuse 20 und damit auch von der Serviceöffnung 8 weggenommen ist, wodurch die Serviceöffnung 8 freigegeben wird und das Tauchmotorrührgerät 23 durch entsprechende Betätigung der Höhenverstellvorrichtung, die hier beispielhaft durch die Handkurbel 24, die Seiltrommel 21 sowie das Seil 22 gebildet ist, so nach oben außerhalb des Fermenterbehälters verfahren werden kann, dass das Tauchmotorrührgerät 23 dort, wie in der Fig. 2 dargestellt, von der Podestplatte 5 ausgehend für Wartungs- und Servicearbeiten frei zugänglich ist.

Um zu verhindern, dass bei einem in der Fig. 1 lediglich beispielhaft eingezeichneten Füllstand H₁ des im Fermenterbehälter 1 aufgenommenen, flüssigen sowie zu vergärenden Substrates 32 das Gas aus der Gasphase 33 über die Serviceöffnung 8 in die Umgebung entweichen kann, weist die erfindungsgemäße Vorrichtung weiter wenigstens einen hier lediglich beispielhaft dargestellten Sensor 34 als Bestandteil einer Füllstands-Messeinrichtung auf. Mittels diesem wenigstens einen Sensor 34 kann die Höhe H₁ des Füllstandes des Substrates 32 messtechnisch erfasst und als Höhenistwert-Signal, wie dies in der Fig. 1 lediglich schematisch und strichliert eingezeichnet ist, einer Steuereinrichtung 35 zugeführt werden, die bei einem im Beispielfall der Fig. 1 erfassten zu geringen Füllstand H₁ des Substrates 32, bei dem der Kragen 16 nicht in das Substrat 32 eintaucht, eine Abpumpeinrichtung 36 für eine definierte, vorgegebene und/oder errechnete Zeitdauer solange abschaltet, dass mittels dieser Abpumpeinrichtung 36 kein flüssiges Substrat mehr über die Rohrleitungen 37, 38, beispielsweise zu einem nachgeschalteten Fermenterbehälter, abgepumpt wird.

Durch dieses fehlende Abpumpen von flüssigem Substrat 32 steigt der Füllstand des Substrates 32 an, zum Beispiel auf die in der Fig. 2 dargestellte Füllstandshöhe H₂, bei der der Kragen 16 der Podestplatte 5 eine vorgegebene Wegstrecke in das Substrat 32 eingetaucht ist und somit den von dem Kragen 16 ringförmig umschlossenen Gasphasenbereich 39 unterhalb der Serviceöffnung 8 sowie oberhalb des Substrates 32 gasdicht von dem restlichen Gasphasenbereich 40 des Fermenterbehälter-Innenraums 14 abtrennt.

Erst dann, wenn der Füllstand des Substrates 32 so weit angestiegen ist, dass dieser in der Fig. 2 gezeigte eingetauchte Zustand des Kragens 16 erzielt ist, wird mittels der Höhenverstellvorrichtung das Tauchmotorrührgerät in die in der Fig. 2 gezeigte Position außerhalb des Fermenterbehälters 1 verfahren bzw. bewegt und können Service- bzw. Wartungsarbeiten am Tauchmotorrührgerät 23 in der erwünschten Weise ohne nennenswerte Gasbelastung durch die Gasphase 40 im Innenraum 14 des Fermenterbehälters 1 durchgeführt werden.

Nach Beendigung der Service- bzw. Wartungsarbeiten kann dann das Tauchmotorrührgerät wieder über die Serviceöffnung 8 in den Innenraum 14 des Fermenterbehälters bewegt werden und anschließend die in der Fig. 2 geöffnete Serviceöffnung 8 bzw. Wartungsöffnung 30 wiederum mittels der Abdeckplatte 27 gasdicht verschlossen werden.

Anschließend kann dann weiter die Steuereinrichtung 35 wiederum die ausgeschaltete Abpumpeinrichtung 36 einschalten und das Abpumpen von flüssigem Substrat 32 über die Rohrleitungen 37, 38 einleiten, wodurch der Füllstand im Fermenterbehälter-Innenraum 14 bevorzugt wiederum auf ein Niveau abfällt, in dem der Kragen 16 nicht in das Substrat 32 eintaucht (vergleiche Fig. 1), wodurch sichergestellt ist, dass wiederum eine einheitliche Gasphase 33 oberhalb des Flüssigkeitsspiegels des Substrates vorhanden ist.

Ersichtlich lassen sich somit mit der erfindungsgemäßen Lösung Wartungs-und Servicearbeiten an einem Tauchgerät, hier an einem beispielhaften Tauchmotorrührgerät 23, funktionssicher und ohne unerwünschte Gasbelastung der Monteure durchführen.

Der Sensor 34 ist hier Bestandteil einer Füllstands-Messvorrichtung, die wenigstens einen derartigen Sensor aufweist, mittels dem der aktuelle Füllstand des Substrates 32 im Fermenterbehälter 1 erfasst und als Höhen-Istwert-Signal der Steuereinrichtung zugeführt wird. Die Steuereinrichtung 35 errechnet dann wenigstens aus diesem Höhen-Istwert-Signal und dem vorgegebenen, bekannten Wert für die Einragtiefe (h) des Kragens in den Fermenterbehälter-Innenraum, ob der Kragen 16 in das Substrat 32 eintaucht oder nicht, wobei die Steuereinrichtung 35 bei einem ermittelten, zu geringen Füllstand, bei dem der Kragen 16 nicht in das Substrat eintaucht, wie dies im Beispielfall in Verbindung mit der Fig. 1 der Fall war, wenigstens eine Förder- und/oder Pumpeinrichtung solange ansteuert, bis der wenigstens eine Sensor 34 ein Höhen-Istwert-Signal liefert, das einem Füllstands-Sollwert und damit einem Füllstand des Substrates 32 im Fermenterbehälter 1 entspricht, bei dem der Kragen 16 in der in der Fig. 2 gezeigten Weise in das Substrat 32 eintaucht.

## Patentansprüche

1. Biogasanlagen-Fermenterbehälter (1) mit einer Serviceeinrichtung, die eine mittels einer Abdeckeinrichtung (20, 27) gasdicht verschließbare, deckenseitige Serviceöffnung (8) aufweist, durch die ein an einem Führungsmast (17) höhenverstellbar geführtes Tauchgerät (23) für Wartungs- und Servicearbeiten im Wesentlichen gasdicht aus dem Fermenterbehälter (1) heraus und wieder in den Fermenterbehälter (1) hinein bewegbar ist,
wobei um die Serviceöffnung herum (8) ein in der Dichtstellung schachtartig nach unten in den Fermenterbehälter-Innenraum (14) einragendes Dichtelement angeordnet ist, das wenigstens bei Wartungs-und Servicearbeiten in das im Fermenterbehälter (1) aufgenommene, zu vergärende flüssige Substrat (32) eintaucht und den von dem Dichtelement ringförmig umschlossenen Gasphasenbereich (39) unterhalb der öffenbaren Serviceöffnung (8) sowie oberhalb des Substrates (32) gasdicht von dem restlichen Gasphasenbereich (40) des Fermenterbehälter-Innenraums (14) abtrennt,
wobei das Dichtelement durch einen starren, nicht verlagerbaren und unflexiblen sowie von der Serviceöffnung (8) dauerhaft in Richtung Fermenterbehälter-Innenraum (14) abragenden rohrstutzenartigen Kragen (16) gebildet ist,
wobei die mittels der Abdeckeinrichtung (20, 27) gasdicht verschließbare, deckenseitige Serviceöffnung (8) Bestandteil einer deckenseitig angeordneten, begehbaren Podestplatte (5) ist, von der der die Serviceöffnung (8) ringförmig umschliessende Kragen (16) nach unten abragt,
**dadurch gekennzeichnet,**
**dass** sich an die aus einem Metall hergestellte Podestplatte (5) eine Behälterwand (3) und ein Foliendach (4) des Fermenterbehälters (1) anschließt,
**dass** die Podestplatte (5) wenigstens einen einer Behälterwand (3) zugeordneten Podestplatten-Randkantenbereich (6) aufweist, der in einer gasdichten Anlageverbindung an einer Behälterseitenwand anliegt und dort fest angebunden ist,
**dass** die Podestplatte (5) einen Foliendach-Anschlussbereich (12) aufweist, an dem das Foliendach (4) gasdicht angebunden ist, und
**dass** die Podestplatte (5) zudem mittels einer Abstützeinrichtung (10) von unten her abgestützt ist.

2. Biogasanlagen-Fermenterbehälter nach Anspruch 1, **dadurch gekennzeichnet, dass** der Serviceeinrichtung weiter eine Füllstands-Messeinrichtung (34) zugeordnet ist oder eine solche aufweist, mittels der wenigstens bei Wartungs- und Servicearbeiten der Füllstand des Substrates (32) im Fermenterbehälter (1) erfasst und ein entsprechendes Füllstands-Messsignal als Höhen-Istwertsignal erzeugt wird, und
dass eine Steuereinrichtung (35) vorgesehen ist, der das Füllstands-Messsignal zugeführt wird und die bei einem erfassten geringen Füllstand, bei dem der Kragen (16) nicht in das Substrat (32) eintaucht, wenigstens eine Förder- und/oder Pumpeinrichtung (36) dergestalt ansteuert, dass der Füllstand des Substrates (32) im Fermenterbehälter (1) auf ein Niveau angehoben wird, bei dem der Kragen (16) in das Substrat (32) eintaucht.

3. Biogasanlagen-Fermenterbehälter nach Anspruch 2, **dadurch gekennzeichnet, dass** die Füllstands-Messvorrichtung wenigstens einen Sensor (34) aufweist, mittels dem der aktuelle Füllstand des Substrates (32) im Fermenterbehälter (1) erfasst und als Höhen-Istwertsignal der Steuereinrichtung (35) zugeführt wird, die wenigstens aus diesem Höhen-Istwertsignal und dem vorgegebenen, bekannten Wert für die Einragtiefe (h) des Kragens (16) in den Fermenterbehälter-Innenraum (14) ermittelt, ob der Kragen (16) in das Substrat (32) eintaucht, wobei die Steuereinrichtung (35) bei einem ermittelten zu geringen Füllstand, bei dem der Kragen (16) nicht in das Substrat (32) eintaucht, die wenigstens eine Förder- und/oder Pumpeinrichtung (36) solange ansteuert, bis der wenigstens eine Sensor (34) ein Höhen-Istwertsignal liefert, das einem Füllstands-Sollwert entspricht und/oder bis ein Füllstand des Substrates im Fermenterbehälter erreicht ist, bei dem der Kragen (16) in das Substrat (32) eintaucht.

4. Biogasanlagen-Fermenterbehälter nach Anspruch 2 oder 3, **dadurch gekennzeichnet, dass** die wenigstens eine Förder- und/oder Pumpeinrichtung durch wenigstens eine, das zu vergärende Substrat (32) aus dem Fermenterbehälter (1) abpumpende Abpumpeinrichtung (36) gebildet ist, die zur Anhebung des Füllstandes von der Steuereinrichtung (35) für eine definierte Zeitdauer abgeschalten wird.

5. Biogasanlagen-Fermenterbehälter nach einem der Ansprüche 2 bis 4, **dadurch gekennzeichnet, dass** die wenigstens eine Förder- und/oder Pumpeinrichtung durch wenigstens eine, zu vergärendes Substrat in den Fermenterbehälter fördernde Pumpeinrichtung gebildet ist, die zur Anhebung des Füllstandes, gesteuert von der Steuereinrichtung, flüssiges Substrat aus einem vor- und/oder nachgeschalteten Behälter in den Fermenterbehälter pumpt.

6. Biogasanlagen-Fermenterbehälter nach einem der vorhergehenden Ansprüche , **dadurch gekennzeichnet, dass** der Kragen (16) durch ein separates Bauteil gebildet ist, das mit der Podestplatte (5) verbunden ist, insbesondere kraft- und/oder stoffschlüssig verbunden ist, oder dass der Kragen (16) integraler Bestandteil der Podestplatte (5) ist und mit dieser materialeinheitlich und/oder einstückig verbunden ist, insbesondere durch aus der Podestplatte (5) herausgebogene Laschen gebildet ist.

7. Biogasanlagen-Fermenterbehälter nach einem der vorhergehenden Ansprüche, **dadurch gekennzeichnet, dass** der Podestplatten-Randkantenbereich (6) mittels einer Schraubverbindung (7) an der Behälterseitenwand (3) fest angebunden ist.

8. Biogasanlagen-Fermenterbehälter nach einem der vorhergehenden Ansprüche, **dadurch gekennzeichnet, dass** das Foliendach (4), insbesondere ein Foliendach-Randbereich, mittels einer Klemmverbindung gasdicht zwischen einem Klemmbereich (11), insbesondere einem Randflansch, der Podestplatte (5) und einer Klemmleiste (15), die mit dem Klemmbereich (11) lösbar verbindbar ist, insbesondere verklemmt ist, und
dass die Abstützeinrichtung (10) wandseitig am Fermenterbehälter-Innenraumwandbereich angebunden und festgelegt ist.

9. Biogasanlagen-Fermenterbehälter nach einem der vorhergehenden Ansprüche, **dadurch gekennzeichnet, dass** die die Serviceöffnung (8) gasdicht verschließende Abdeckeinrichtung durch wenigstens einen Deckel (27) und/oder durch ein von der Serviceöffnung weg nach oben ragendes Gehäuse (20), das einen Aufnahmeraum für wenigstens einen oberen Teilbereich des Führungsmastes (17) ausbildet, gebildet ist.

10. Biogasanlagen-Fermenterbehälter nach einem der vorhergehenden Ansprüche, **dadurch gekennzeichnet, dass** die Podestplatte (5) in etwa mittig und zentral die Serviceöffnung (8) aufweist, an die sich zu beiden Seiten hin flügelartige Podestplattenbereiche (9a, 9b) anschließen.

## Claims

1. Biogas-plant fermenter tank (1) having a service arrangement which has a ceiling-level service opening (8) which can be closed in a gas-tight manner by means of a covering device (20, 27) and through which a submersible apparatus (23), which is guided in a height-adjustable manner on a guide pole (17), can be moved in an essentially gas-tight manner out of the fermenter tank (1), for maintenance and servicing work, and back into the fermenter tank (1) again,
wherein the service opening (8) has arranged around it a sealing element which, in the sealing position, projects downwards in a shaft-like manner into the fermenter-tank interior (14) and, at least when maintenance and servicing work is being carried out, penetrates into the fermentable liquid substrate (32) accommodated in the fermenter tank (1) and, in a gas-tight manner, separates the gas-phase region (39) beneath the openable service opening (8) and above the substrate (32), said gas-phase region being enclosed in annular form by the sealing element, from the rest of the gas-phase region (40) of the fermenter-tank interior (14),
wherein the sealing element is formed by a rigid, non-displaceable and inflexible tubular-stub-like collar (16) which projects permanently from the service opening (8) in the direction of the fermenter-tank interior (14), wherein the ceiling-level service opening (8), which can be closed in a gas-tight manner by means of the covering device (20, 27), is a constituent part of a walk-on platform panel (5), which is arranged at ceiling level and from which the collar (16), which encloses the service opening (8) in annular form, projects downwards,
**characterized**
**in that** a tank wall (3) and a sheet-material roof (4) of the fermenter tank (1) adjoin the platform panel (5), which is produced from a metal,
**in that** the platform panel (5) has at least one peripheral-edge region (6), which is assigned to a tank wall (3) and butts, by way of a gas-tight connection, against a side wall of the tank and is firmly attached there,
**in that** the platform panel (5) has a connection region (12) for the sheet-material roof, the sheet-material roof (4) being attached in a gas-tight manner to said connection region, and
**in that** the platform panel (5), in addition, is supported from beneath by means of a supporting device (10).

2. Biogas-plant fermenter tank according to Claim 1, **characterized in that** the service device is also assigned a filling-level-measuring device (34), or has one such, by means of which, at least when maintenance and servicing work is being carried out, the filling level of the substrate (32) in the fermenter tank (1) is sensed and a corresponding filling-level-measurement signal is generated in the form of an actual-height-value signal, and
**in that** a control device (35) is provided, and this control device is fed the filling-level-measurement signal and, in the case of a low filling level at which the collar (16) does not penetrate into the substrate (32) being sensed, activates at least one delivery and/or pumping device (36) such that the filling level of the substrate (32) in the fermenter tank (1) is raised to a level at which the collar (16) penetrates into the substrate (32).

3. Biogas-plant fermenter tank according to Claim 2, **characterized in that** the filling-level-measuring device has at least one sensor (34) by means of which the current filling level of the substrate (32) in the fermenter tank (1) is sensed and is fed, in the form of an actual-height-value signal, to the control device (35), which uses at least this actual-height-value signal and the predetermined, known value for the depth (h) to which the collar (16) projects into the fermenter-tank interior (14) to determine whether the collar (16) penetrates into the substrate (32), wherein, in the case of an excessively low filling level at which the collar (16) does not penetrate into the substrate (32) being determined, the control device (35) activates the at least one delivery and/or pumping device (36) until the at least one sensor (34) delivers an actual-height-value signal which corresponds to a desired filling-level value and/or until the filling level of the substrate in the fermenter tank achieved is one at which the collar (16) penetrates into the substrate (32).

4. Biogas-plant fermenter tank according to Claim 2 or 3, **characterized in that** the at least one delivery and/or pumping device is formed by at least one pumping-out device (36) which pumps the fermentable substrate (32) out of the fermenter tank (1) and, in order for the filling level to be raised, is switched off by the control device (35) for a defined period of time.

5. Biogas-plant fermenter tank according to one of Claims 2 to 4, **characterized in that** the at least one delivery and/or pumping device is formed by at least one pumping device which delivers fermentable substrate into the fermenter tank and, in order for the filling level to be raised, with control provided by the control device, pumps liquid substrate into the fermenter tank from an upstream and/or downstream tank.

6. Biogas-plant fermenter tank according to one of the preceding claims, **characterized in that** the collar (16) is formed by a separate component which is connected to the platform panel (5), in particular is connected thereto in a force-fitting manner and/or with material bonding, or **in that** the collar (16) is an integral constituent part of the platform panel (5) and is connected integrally to the latter and/or in one piece, in particular is formed by lugs bent out of the platform panel (5).

7. Biogas-plant fermenter tank according to one of the preceding claims, **characterized in that** the peripheral-edge region (6) of the platform panel is firmly attached to the side wall (3) of the tank by means of a screw connection (7).

8. Biogas-plant fermenter tank according to one of the preceding claims, **characterized in that** the sheet-material roof (4), in particular a peripheral region of the sheet-material roof, is in particular clamped in a gas-tight manner, by means of a clamping connection, between a clamping region (11), in particular a peripheral flange, of the platform panel (5) and a clamping bar (15), which can be connected in a releasable manner to the clamping region (11), and
**in that** the supporting device (10) is attached, and secured, to the wall in the interior wall region of the fermenter tank.

9. Biogas-plant fermenter tank according to one of the preceding claims, **characterized in that** the covering device, which closes the service opening (8) in a gas-tight manner, is formed by at least one cover (27) and/or by a housing (20), which projects upwards away from the service opening and forms an accommodating space for at least one upper subregion of the guide pole (17).

10. Biogas-plant fermenter tank according to one of the preceding claims, **characterized in that** the service opening (8) is approximately in the middle, and in the centre, of the platform panel (5) and is adjoined on either side by wing-like platform-panel regions (9a, 9b).

## Revendications

1. Cuve de fermentation (1) pour installations de biogaz avec un mécanisme d'entretien qui présente une ouverture de maintenance (8), laquelle se trouve du côté du plafond et laquelle peut être obturée de manière hermétique aux gaz au moyen d'un mécanisme de recouvrement (20, 27), à travers laquelle ouverture de maintenance une cuve à immersion (23), guidée de manière réglable en hauteur au niveau d'un mât de guidage (17), peut être déplacée, pour l'essentiel de manière hermétique aux gaz, en dehors de la cuve de fermentation (1) pour des travaux d'entretien et de maintenance et peut être réinsérée dans la cuve de fermentation (1) ;
dans laquelle un élément d'étanchéité qui pénètre vers le bas, en prenant la forme d'une cheminée, dans la chambre intérieure (14) de la cuve de fermentation, quand ledit élément d'étanchéité se trouve dans la position étanche, est disposé tout autour de l'ouverture de maintenance (8), lequel élément d'étanchéité est immergé, tout au moins lors des travaux d'entretien et de maintenance, dans le substrat (32) liquide qui est collecté et destiné à être fermenté dans la cuve de fermentation (1) ; et
dans laquelle ledit élément d'étanchéité sépare, de manière hermétique aux gaz, la zone de phase gazeuse (39), laquelle est entourée de manière circulaire par l'élément d'étanchéité, au-dessous de l'ouverture de maintenance (8) pouvant être ouverte, ainsi qu'au-dessus du substrat (32), du reste de la zone de phase gazeuse (40) de la chambre intérieure (14) de la cuve de fermentation ;
dans laquelle l'élément d'étanchéité est formé par une collerette (16) rigide et inflexible, en forme de tubulure, laquelle ne peut pas être déplacée et laquelle fait saillie en permanence depuis l'ouverture de maintenance (8) en direction de la chambre intérieure (14) de la cuve de fermentation ;
dans laquelle l'ouverture de maintenance (8), laquelle se situe du côté du plafond et laquelle peut être obturée de manière hermétique aux gaz au moyen du mécanisme de recouvrement (20, 27), fait partie intégrante d'une plaque de palier (5), laquelle est praticable et disposée du côté du plafond, et depuis laquelle la collerette (16) qui entoure l'ouverture de maintenance (8) de manière circulaire fait saillie vers le bas ;
**caractérisée en ce qu'**une cloison (3) de la cuve et une toiture en film (4) de la cuve de fermentation (1) viennent se raccorder à la plaque de palier (5) fabriquée à partir d'un métal ;
**caractérisée en ce que** la plaque de palier (5) présente tout au moins une zone de bordure périphérique (6) de la plaque de palier associé à une cloison (3) de la cuve, laquelle zone de bordure périphérique prend appui sur une cloison latérale de la cuve, dans une jonction par prise d'appui, hermétique aux gaz, et y est solidement fixée ;
**caractérisée en ce que** la plaque de palier (5) présente une zone de raccordement (12) de la toiture en film, à laquelle est fixée la toiture en film (4) de manière hermétique aux gaz ; et
**caractérisée en ce que** la plaque de palier (5) est en outre soutenue par le bas au moyen d'un mécanisme de soutien (10).

2. Cuve de fermentation pour installations de biogaz selon la revendication 1 ;
**caractérisée en ce qu'**un mécanisme de mesure du niveau de remplissage (34) est par ailleurs associé au mécanisme d'entretien ou **caractérisée en ce que** le mécanisme d'entretien présente un tel mécanisme de mesure du niveau de remplissage, au moyen duquel le niveau de remplissage du substrat (32) se trouvant dans la cuve de fermentation (1) est détecté, tout au moins lors des travaux d'entretien et de maintenance, et un signal de mesure correspondant du niveau de remplissage est généré sous la forme d'un signal de valeur réelle de la hauteur de remplissage ; et
**caractérisée en ce qu'**est prévu un mécanisme de commande (35) vers lequel est acheminé le signal de mesure du niveau de remplissage et lequel mécanisme de commande pilote tout au moins un mécanisme de transport et/ou de pompage (36), quand un niveau de remplissage trop bas est détecté, à partir duquel la collerette (16) n'est plus immergée dans le substrat (32), de telle sorte que le niveau de remplissage du substrat (32) dans la cuve de fermentation (1) est augmenté à un niveau, à partir duquel la collerette (16) est de nouveau immergée dans le substrat (32).

3. Cuve de fermentation pour installations de biogaz selon la revendication 2,
**caractérisée en ce que** le dispositif de mesure du niveau de remplissages présente tout au moins une sonde (34), au moyen de laquelle le niveau de remplissage actuel du substrat (32) dans la cuve de fermentation (1) est détecté et est acheminé vers le mécanisme de commande (35) sous la forme d'un signal de valeur réelle de la hauteur de remplissage, lequel mécanisme de commande détermine, tout au moins à partir de ce signal de valeur réelle de la hauteur de remplissage, ainsi qu'à partir de la valeur connue, prédéterminée pour la profondeur de pénétration (h) de la collerette (16) dans la chambre intérieure (14) de la cuve de fermentation, si ladite collerette (16) est immergée dans le substrat (32) ;
dans laquelle le mécanisme de commande (35) pilote le tout au moins un mécanisme de transport et/ou de pompage (36), quand un niveau de remplissage trop bas a été déterminé, à partir duquel la collerette (16) n'est plus immergée dans le substrat (32), jusqu'à ce que la tout au moins une sonde (34) fournisse un signal de valeur réelle de la hauteur de remplissage qui corresponde à une valeur de consigne du niveau de remplissage et/ou jusqu'à ce qu'un niveau de remplissage du substrat soit atteint dans la cuve de fermentation, à partir duquel la collerette (16) est de nouveau immergée dans le substrat (32).

4. Cuve de fermentation pour installations de biogaz selon la revendication 2 ou 3,
**caractérisée en ce que** le tout au moins un mécanisme de transport et/ou de pompage est formé par tout au moins un mécanisme d'évacuation par pompage (36), lequel pompe le substrat (32) devant être fermenté en dehors de la cuve de fermentation (1) et lequel est déconnecté pour une période de temps définie par le mécanisme de commande (35), en vue de l'augmentation du niveau de remplissage.

5. Cuve de fermentation pour installations de biogaz selon l'une des revendications 2 à 4,
**caractérisée en ce que** le tout au moins un mécanisme de transport et/ou de pompage est formé par tout au moins un mécanisme de pompage, lequel transporte le substrat devant être fermenté dans la cuve de fermentation et lequel pompe le substrat liquide dans la cuve de fermentation à partir d'une cuve se trouvant en aval et/ou en amont, sous le pilotage du mécanisme de commande, en vue de l'augmentation du niveau de remplissage.

6. Cuve de fermentation pour installations de biogaz selon l'une des revendications précédentes,
**caractérisée en ce que** la collerette (16) est formée par un élément de structure séparé qui est relié à la plaque de palier (5), en particulier qui est relié par association de force et/ou par association de matière ; ou
**caractérisée en ce que** la collerette (16) fait partie intégrante, dans son intégralité, de la plaque de palier (5) et est reliée avec cette dernière en une seule matière et/ou d'une seule pièce, en particulier est formée par des languettes recourbées à partir de la plaque de palier (5).

7. Cuve de fermentation pour installations de biogaz selon l'une des revendications précédentes,
**caractérisée en ce que** la zone de la bordure périphérique (6) de la plaque de palier est solidement fixée à la cloison latérale de la cuve (3) au moyen d'un raccord à vis (7).

8. Cuve de fermentation pour installations de biogaz selon l'une des revendications précédentes,
**caractérisée en ce que** la toiture en film (4), en particulier une zone de la bordure de la toiture en film, peut être raccordée, en particulier est bloquée, de manière amovible et hermétique aux gaz, avec une zone de serrage (11), au moyen d'une liaison par serrage qui est située entre ladite zone de serrage (11), en particulier entre une bride de la bordure, la plaque de palier (5) et une barrette de raccordement (15) ; et
**caractérisée en ce que** le mécanisme de soutien (10) est fixé et positionné du côté de la cloison, au niveau de la zone de la cloison de la chambre intérieure de la cuve de fermentation.

9. Cuve de fermentation pour installations de biogaz selon l'une des revendications précédentes,
**caractérisée en ce que** le mécanisme de recouvrement qui obture l'ouverture de maintenance (8) de manière hermétique aux gaz est formé par tout au moins un couvercle (27) et/ou par un boîtier (20) qui fait saillie de l'ouverture de maintenance en s'étendant vers le haut, lequel boîtier forme un compartiment de réception pour tout au moins une zone partielle supérieure du mât de guidage (17).

10. Cuve de fermentation pour installations de biogaz selon l'une des revendications précédentes,
**caractérisée en ce que** la plaque de palier (5) présente l'ouverture de maintenance (8) dans une position de manière à peu près médiane et centrale, à laquelle viennent se raccorder, sur les deux côtés, des zones (9a, 9b) en forme d'ailettes de la plaque de palier.
